# EUROPEAN PATENT APPLICATION

(11) **EP 4 763 273 A1**
(43) Date of publication of application: **24.06.2026**
(21) Application number: 25222521.4
(22) Date of filing: 11.12.2025
(51) Int. Cl.: A61N 1/30, A61K 9/00

(54) **DEVICE FOR ELECTRIC STIMULATION OF THE EAR**

(30) Priority: 17.12.2024 EP 24220700
(71) Applicant: Oticon A/S, 2765 Smørum (DK)
(72) Inventor: NAVNTOFT, Charlotte Amalie, DK-2765 Smørum (DK)
(74) Representative: Demant

(57) **Abstract**

The present application relates to a device (300) comprising a first electrode (310) and a second electrode (320), wherein the first electrode (310) is configured to be located in a middle ear space in electrical contact with an inner ear (130), and wherein the second electrode (320) is configured to be located in the middle ear space. The device further comprises a controller for controlling a current generator configured to generate an electrical voltage between the first electrode (310) and the second electrode (320), so as to support delivery of a substance (200), provided in the middle ear space, into the inner ear (130).

## Description

### FIELD

The present disclosure relates to hearing therapeutics. More particularly, the disclosure inter alia relates to non-invasive methods of supporting deployment or delivery of a substance, such as a drug or pharmaceutical solutions into the inner ear.

### BACKGROUND

Hearing therapeutics is an emerging area that seeks to develop pharmaceutical solutions for the inner ear to prevent or even cure hearing loss. A key facet of a therapeutic effect is the requirement for optimal drug exposure in the inner ear for a sufficient amount of time.

Drug delivery to the inner ear is challenging due to several factors. Firstly, a systemic administration of drugs can lead to various side effects throughout the body, especially if high drug doses are required. By using trans-tympanic administration of the drug into the middle ear space, the drug can be localized to the inner ear, minimizing the risk of systemic side effects. This targeted drug delivery approach enhances the therapeutic benefit while reducing the potential for adverse reactions in other organs or tissues.

However, the inner ear is a highly specialized and intricate structure located deep within the skull. It is protected by several barriers that make it difficult for drugs to reach their target site. Many inner ear disorders may require long-term treatment, and patient compliance can be an issue. The need for repeated administration can be burdensome and require a minimal invasiveness procedure. A direct administration of drugs into the inner ear via penetration of the inner ear is thus often not feasible.

Furthermore, the inner ear is a highly individualized structure, and variations in its anatomy and physiology can affect drug concentrations and response. Personalized drug delivery approaches may be required to account for these variations and optimize treatment outcomes. Also, some drugs may degrade or lose their efficacy when exposed to the unique environment of the inner ear. Factors such as pH, temperature and enzymatic activity can affect the stability of the drugs, making it challenging to maintain their therapeutic concentration.

Therefore, there is a need to provide a solution that allows for an enhanced drug administration to the inner ear. The present disclosure provides an improvement with respect to at least some aspects over the prior art.

### SUMMARY

According to a first aspect, a method for supporting delivery of a substance to an inner ear is provided. The method may comprise providing at least one first (e.g. a stimulation) electrode in a middle ear space. The at least one first electrode may be provided close to and/or in electrical contact with the inner ear. The method may comprise providing at least one second (e.g. a return) electrode (e.g. in the middle ear space, as well). The method may comprise providing an electrical voltage between the at least one first electrode and the at least one second electrode, so as to support delivery of the substance, provided in the middle ear space, into the inner ear. The method may also comprise providing the substance to the middle ear space.

According to a second aspect, a device is provided. The device may be configured to perform a method according to the first aspect. The device may comprise at least one first (e.g. stimulation) electrode. The device may comprise at least one second (e.g. return) electrode. The device may comprise means to provide the at least one first electrode in the middle ear space close to and/or in electrical contact with the inner ear. The device may comprise means to provide the at least one second electrode (e.g. in the middle ear space, as well). The device may comprise means to provide an electrical voltage between the at least one first electrode and the at least one second electrode, so as to support delivery of a substance, provided in the middle ear space, into the inner ear.

According to a third aspect, a system is provided. The system may in particular be configured to perform a method for supporting delivery of a substance to an inner ear, in particular according to the first aspect. The system may in particular be provided for the use in a method for supporting delivery of a substance to an inner ear, in particular according to the first aspect. The system may comprise the device according to the second aspect. The system may comprise means for providing a substance into a middle ear space.

Method steps disclosed below may disclose features of a device or system configured to perform said method steps. Disclosed method steps providing a device or system feature may also disclose the corresponding device or system feature. Accordingly, a disclosed device or system feature configured to perform a method step may also disclose a corresponding method step.

The inner ear may be a component of a human ear or a component of an animal ear. The inner ear may comprise a cochlea and vestibular system organs. Thus, the inner ear may be a part of a human body or an animal body that may transform audio signals that were registered by a corresponding tympanic membrane into nerve signals.

The substance (which may also be referred to as substance of interest herein) may in particular be a drug or therapeutics, in particular a hearing therapeutics. Thus, the drug may have to be provided to the inner ear to achieve a desired effect of the drug. For example, the drug may be provided to prevent or cure hearing loss of a patient. Various approaches to deliver the drug to the inner ear may include solution-based methods, hydrogel-based methods, nano-based methods, or microneedles. Although each of these approaches may have advantages and limitations, none may offer delivery of therapeutic relevant drug levels to the inner ear in a safe and long-term effective manner.

The round window membrane and oval window membrane are non-osseous barriers that separate the middle and inner ear compartments. Substances that are administered into the middle ear space, for example via trans-tympanic administration (e.g. by penetrating a corresponding tympanic membrane with a syringe comprising the substance), may penetrate the round window membrane and/or the oval window membrane to enter the inner ear. In other words, the round window membrane and/or the oval window membrane may be ideal portals for topically delivering therapeutics to the inner ear via trans-tympanic administration into the middle ear space. However, some substances may have limited penetration from the middle ear into the inner ear through the round window membrane and/or through the oval window membrane, e.g. when administered through traditional trans-tympanic administration routes. For example, certain antibiotics may not adequately reach the cochlea, where they may be needed to treat infections like labyrinthitis. Another example is neurotrophic or regenerative factors, such as growth factors, may not adequately reach the cochlea to protect or regenerate inner ear structures, respectively.

According to the first aspect, at least one first electrode and optionally at least one second electrode are provided in the middle ear space. It was found that by providing the electrical voltage between the at least one first electrode and the at least one second electrode, so as to support delivery of a substance, provided in the middle ear space, into the inner ear, a penetration of e.g. the round window membrane and/or the oval window membrane by the substance may be improved. The voltage (and thus the current) may be specifically adjusted and/or tailored with respect to the substance of interest (and/or the electrically conductive substance carrying the substance of interest), i.e. in particular the electrical properties thereof, so as to support the passing of the substance of interest into the middle ear. The described approach may utilize the process of iontophoresis. Thus, providing the electrical voltage may enable a more efficient transfer of the substance of interest from the middle ear to the inner ear. This is because the voltage may allow for increasing the permeability of a membrane of the inner ear or between the inner ear and the middle ear (such as round window membrane and/or the oval window membrane) with an electric current. Further, the voltage may allow for a driving force to move the substance of interest, such as a drug, through the so permeabilized membrane. As will be explained in more detail below, the voltage and the current between respective electrodes may be controlled (e.g. by a current generator and controller) depending on the specific substance and its properties to be delivered. Further, it may even be possible that by providing the electrical voltage, substances may penetrate the round window membrane and/or the oval window membrane which could not penetrate the round window membrane and/or the oval window membrane without providing the electrical voltage. Thus, the method according to the first aspect allows for an improved non-invasive drug administration to the inner ear.

The at least one first electrode may in particular be at least one stimulating electrode. The at least on second electrode may in particular be at least on return electrode.

As will be explained in more detail below, the substance itself may consist of or comprise electrically charged molecules. Additionally or alternatively, the substance of interest may be solved in an electrically conductive (carrier) substance (such as an electrolyte), which may drag the substance of interest along.

The device and/or the system, may further comprise a ground (or earth) electrode.

The substance of interest may be provided in an electrically conductive (carrier) substance. The electrically conductive substance may enhance transport of the molecules of interest into the inner ear (e.g. across oval window membrane and/or round window membrane). The electrically conductive substance may further enable an electrical connection between the at least one first electrode and a structure of the inner ear, such as the round window membrane and/or the oval window membrane. In particular, the electrically conductive substance may allow for an electrical current to flow between the at least one first electrode and the structure of the inner ear. The electrical current may, for example, flow through the round window membrane and/or the oval window membrane thus enhancing a permeability of the round window membrane and/or the oval window membrane for particles such as drug particles.

The electrically conductive substance may be or comprise a conductive gel or a conductive solution. Providing the substance of interest in a conductive gel or a conductive solution allows for an easier deployment of the substance of interest in the middle ear. In particular, after providing the substance of interest in the middle ear, for example in direct contact with the round window membrane and/or the oval window membrane, it may be desirable that the substance of interest remains where it was provided. By providing the substance of interest in a conductive gel or a conductive solution, the substance of interest may remain at the location where it was provided for a longer period of time.

As described above, the electrically conductive substance may be provided in contact with a component of the middle ear space. In particular, the electrically conductive substance may be provided in contact with the round window membrane and/or the oval window membrane. Thus, a transfer of the substance of interest from the electrically conductive substance to the inner ear through the round window membrane and/or the oval window membrane may be enabled.

Further, the electrically conductive substance may be arranged at least in part between the inner ear and the at least one first electrode. In other words, the at least one first electrode may be arranged such that at least part of the electrically conductive substance is arranged in between the inner ear and the at least one first electrode. In particular, the electrically conductive substance may be arranged at least in part between the round window membrane and the at least one first electrode and/or at least in part between the oval window membrane and the at least one first electrode. With the electrically conductive substance being arranged at least in part between the inner ear and the at least one first electrode, the substance of interest may be provided in contact with the round window membrane and/or the oval window membrane while simultaneously allowing for the at least one first electrode to be provided close to the round window membrane and/or the oval window membrane.

As mentioned above, the at least one first electrode is provided in the middle ear space close to and/or in electrical contact with the inner ear. Thereby, being close to the inner ear may be understood as at least partially physically touching a component of the inner ear. Being close to the inner ear may be understood as at least partially being separated from the inner ear by a distance not more than 5 mm, in particular not more than 3mm, in particular not more than 2 mm.

Being in electrical contact with the inner ear may be understood as being in physical contact with a component of the inner ear, such as the round window membrane and/or the oval window membrane. Further, being in electrical contact with the inner ear may be understood as being connected to a component of the inner ear, such as the round window membrane and/or the oval window membrane, by an electrically conductive substance. In particular, being in electrical contact with the inner ear may be understood as being connected to a component of the inner ear, such as the round window membrane and/or the oval window membrane, through the electrically conductive substance.

The at least one first electrode being arranged close to and/or in electrical contact with the inner ear may allow for directing an electrical current induced by the electrical voltage between the at least one first electrode and the at least one second electrode to flow through a component of the inner ear, such as the round window membrane and/or the oval window membrane.

At least one of the at least one first electrode may be provided close to and/or in electrical contact with the round window membrane and/or the oval window membrane of the inner ear. The at least one first electrode may comprise multiple first electrodes. Thereby, it may be sufficient for the method according to the first aspect, if at least one of the at least one first electrode is provided close to and/or in electrical contact with the round window membrane and/or the oval window membrane. In particular, by providing multiple first electrodes, it may be easier to ensure that at least one of the multiple first electrodes is provided close to and/or in electrical contact with the round window membrane and/or the oval window membrane. Thus, an easier and more reliable deployment of the at least one first electrode may be enabled.

At least one of the at least one first electrode may be provided close to and/or in electrical contact with the round window membrane. At least a second one of the at least one first electrode may be provided close to and/or in electrical contact with the oval window membrane. Thus, in particular, at least one of the at least one first electrode may be provided close to and/or in electrical contact with the round window membrane and at least a second one of the at least one first electrode may be provided close to and/or in electrical contact with the oval window membrane, or reverse. Thus, a transfer of the substance of interest may be enhanced simultaneously at the round window membrane and the oval window membrane.

For example, the at least second one of the at least one first electrode may be provided on the promontory (in the middle ear).

For example, the at least second one of the at least one first electrode may be provided in the middle ear, or on the head of the patient.

The at least one second electrode may be provided close to and/or in electrical contact with the inner ear. By providing the at least one second electrode close to and/or in electrical contact with the inner ear, an electrical current can flow from the inner ear through the at least one second electrode. In particular, the electrical voltage may cause an electrical current to pass from the at least one first electrode to the at least one second electrode. Furthermore, the electrical current induced by the electrical voltage may flow from the at least one first electrode to the at least one second electrode through at least part of the inner ear.

In particular, the at least one second electrode may be provided close to and/or in electrical contact with the round window membrane and/or the oval window membrane. An electrical current passing through the at least one second electrode may thus have to flow at least in part through the round window membrane and/or the oval window membrane. Thus, a flow of the electrical current through the round window membrane and/or the oval window membrane may be enhanced.

As mentioned above, the electrical current may pass at least partly through a part of the inner ear. In particular, the electrical current may pass at least partly through a part of the round window membrane and/or a part of the oval window membrane. It was found that by having electrical currents pass through a membrane, it is possible to increase the permeability of said membrane. In other words, electrical currents flowing through a tissue or membrane may enable diffusion of particles through said tissue or membrane.

Thereby, it was found that applying only small currents may often be sufficient and/or beneficial. For example, electrical currents of at least 0.01 mA, preferably at least 0.1 mA may be applied. For example, electrical currents of at most 1 mA, preferably at most 0.5 mA may be applied. Such currents have been found to increase permeability of various membranes and to drive desired substances through the membranes. However, depending on the scenario larger electrical currents may also be used for increasing the permeability of membranes of the inner ear, bordering the inner and middle ear. Furthermore, the electrical current may be induced either as alternating current (AC) or as direct current (DC). The electrical current may also be a pulsed direct current. The electrical current may be induced with varying strength, polarization, duration and/or periodicity.

To provide the electrical current, a current controller and a current generator may be provided. The current controller may control the current generator to provide an electrical current in accordance with above-mentioned criteria regarding strength, polarization, duration and/or periodicity.

Further, the electrical voltage may induce an electrical field between at least one of the at least one first electrode and the round window membrane and/or between at least one of the at least one first electrode and the oval window membrane. Depending on the polarity of the electrical voltage, charged particles may thus be pulled towards the at least one of the at least one first electrode or pushed away from the at least one of the at least one first electrode, depending on the charge of the particles.

The substance may comprise (e.g. drug) particles with an electrical charge. For example, the substance may comprise particles with negative electrical charge and/or with positive electrical charge. The particles may be ionized or undissociated agents. Furthermore, the particles may be ions or other charged atomic compounds. By comprising an electrical charge, said particles may be accelerated through an electrical force, e.g. exerted through an electrical field of an electrode, also without an additional conductive gel or the like.

The electrical voltage may be provided such that the electrical field exerts a force on the particles towards the round window membrane and/or the oval window membrane. In other words, the electrical field may be provided to exert an electrical force on particles arranged between the at least one first electrode and the round window membrane and/or the oval window membrane towards and/or through the round window membrane and/or the oval window membrane. Said electrical force may increase a possibility that said particles transfer from the middle ear space to the inner ear. Thus, applying the electrical voltage may further enhance a delivery of the substance to the inner ear by means of the electrical force applied to charged particles by the electrical field.

The method may further comprise providing a sensor or chamber close to the round window membrane and/or the oval window membrane. The sensor or chamber may be configured to collect, sample and/or detect fluids, in particular fluids from the inner ear. The method may further comprise receiving (and e.g. collecting, sampling and/or detecting) fluids from the inner ear at the sensor or chamber. Said fluids from the inner ear may comprise information about a state of the inner ear. Thus, by collecting, sampling and/or detecting said fluids, said information may be obtained.

To this end, the device may further comprise a sensor configured to collect, sample and/or detect fluids from the inner ear. Alternatively or in addition, the device may further comprise a chamber configured to collect, sample and/or detect fluids from the inner ear. Furthermore, the device may further comprise means to provide the sensor or chamber close to the round window membrane and/or the oval window membrane. Said fluids from the inner ear may pass through the round window membrane and/or the oval window membrane. Thus, said sensor or chamber may be configured to be arranged close to the round window membrane and/or the oval window membrane to efficiently collect, sample and/or detect said fluids.

The device may be provided for the use in a method for supporting delivery of substance to an inner ear. The method for supporting delivery of a substance to an inner ear may be a method according to the first aspect. Thus, the device may be configured to provide all components necessary to perform the method according to the first aspect.

### BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:
- FIG. 1: schematically shows an exemplary method of providing drugs in a middle ear space;
- FIGS. 2a, 2b: schematically show exemplary configurations of stimulation electrodes and return electrodes in a middle ear space; and
- FIGS. 3a-3c: schematically show exemplary devices according to the application.

### DETAILED DESCRIPTION

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the apparatus and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

The electronic hardware may include micro-electronic-mechanical systems (MEMS), integrated circuits (e.g. application specific), microprocessors, microcontrollers, digital signal processors (DSPs), field programmable gate arrays (FPGAs), programmable logic devices (PLDs), gated logic, discrete hardware circuits, printed circuit boards (PCB) (e.g. flexible PCBs), and other suitable hardware configured to perform the various functionality described throughout this disclosure, e.g. sensors, e.g. for sensing and/or registering physical properties of the environment, the device, the user, etc. Computer program shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise.

The described electronic hardware may in particular be used to generate and/or control electrical currents between respective electrodes for increasing the permeability of membranes and utilize a driving force to move the substance or molecule of interest through the permeabilized membrane.

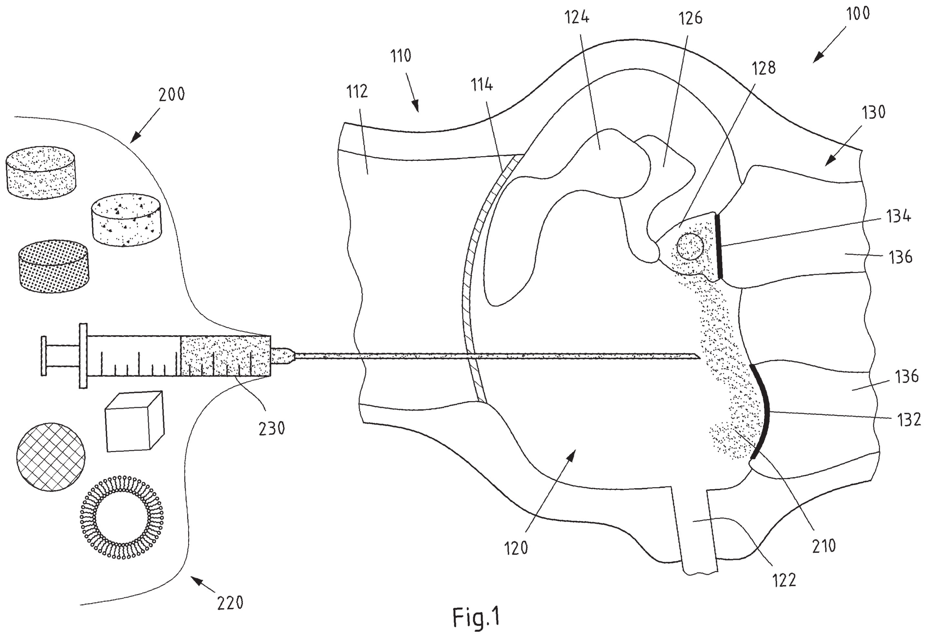

Now referring to Fig. 1, which illustrates a section of an ear 100, for example of a human ear. The ear comprises an outer ear section 110 comprising an ear channel 112 and a tympanic membrane 114. The tympanic membrane 114 separates the outer ear section from a middle ear space 120. The middle ear space 120 comprises an eustachian tube 122 and three bones of the middle ear, a malleus bone 124, an incus bone 126 and a stapes bone 128. The ear 100 further comprises an inner ear section 130 separated from the middle ear space 120 by the round window membrane 132 and the oval window membrane 134. In a healthy ear 100, the stapes bone 128 is typically in contact with the oval window membrane 134.

The inner ear comprises further ear components such as the cochlea 136, which is connected to the round window membrane 132 and the oval window membrane 134. The round window membrane 132 and the oval window membrane 134 are non-osseus barriers, potentially making them suitable portals for topically delivering drugs 200 to the inner ear 130 via trans-tympanic administration of drugs 200 to the middle ear space 120.

Fig. 1 further shows a syringe 230 (an example of means for delivering drugs to the inner ear) injecting an electrically conductive substance 210 comprising the drug 200 and in particular comprising drug particles 220 into the middle ear space. The drug particles may in particular consist of or comprise hydrogels and/or nanoparticles, for instance poloxamer, chitosan, collagen, cubosome, polymeric and/or liposome. Thereby, the syringe pierces through the tympanic membrane. However, it may equally be possible to provide the drug 200 to the middle ear space, e.g. through other tissue of the ear 100 or through the eustachian tube 122. In the present embodiment, the drug 200 is a hearing therapeutics and the exemplarily illustrated drug particles 220 are either at least partially (preferably entirely) negatively charged or at least partially (preferably entirely) positively charged. However, also a mixture of positively and negatively charged drug particles 220 would be possible. In such a case, a respective positively charged particle may be transferred preferably through one of the round window membrane and the oval window membrane while a respective negatively charged particle may be transferred preferably through the other of the round window membrane and the oval window membrane. Additionally or alternatively, the drug particles may be provided in an conductive substance, such as a electrolyte gel.

In Fig. 1, the electrically conductive substance 210 is a conductive gel but may as well be a conductive solution or any other conductive medium that is injectable through a syringe. After being injected into the middle ear space 120 by the syringe 230, the electrically conductive substance 210 is in contact with the round window membrane 132 and the oval window membrane 134. However, it is also possible, that the electrically conductive substance 210 is provided only in contact with one of the round window membrane 132 or the oval window membrane 134. In such a case, the drug would only transfer through a respective one of the round window membrane 132 and the oval window membrane 134 that is in contact with the electrically conductive substance 210.

It is possible that drugs 200 comprised by the electrically conductive substance 210 may transmit through the round window membrane 132 and/or the oval window membrane 134 by osmosis or any other transfer mechanism. However, some drugs 200 may not be compatible for a transfer through the round window membrane 132 and the oval window membrane 134, or a transfer may not be efficient enough to achieve a sufficient level in the inner ear 130.

However, a key facet of a therapeutic effect is the requirement for optimal drug exposure in the inner ear for sufficient amount of time. Various delivery approaches have been trialled, including solution-, hydrogel-, nano-based methods, or microneedles. Although each strategy has advantages and limitations, none offers delivery of therapeutic relevant drug levels in a safe and long-term effective manner. Indeed, there is a need to develop new solutions to effectively deliver drugs to the inner ear.

Drug delivery to the inner ear is in particular challenging due to several factors:
1. Anatomy and barriers: the inner ear is highly specialized and intricate structure located deep within the skull. It is protected by several barriers that make it difficult for drugs to reach their target site. The round window membrane (RWM) and oval window membrane (OWM) are non-osseous barriers that separate the middle and inner ear compartments, potentially making them ideal portals for topically delivering therapeutics to the inner ear via trans-tympanic administration into the middle ear space.
2. Drug stability and degradation: some drugs may degrade or lose their efficacy when exposed to the unique environment of the inner ear. Factors such as pH, temperature and enzymatic activity can affect the stability of the drugs, making it challenging to maintain their therapeutic concentration.
3. Patient compliance: many inner ear disorders may require long-term treatment, and patient compliance can be an issue. The need for repeated administration can be burdensome and require a minimal invasiveness procedure.
4. Individual variations: the inner ear is a highly individualized structure, and variations in its anatomy and physiology can affect drug concentrations and response. Personalized drug delivery approaches may be required to account for these variations and optimize treatment outcomes.

In summary, addressing these challenges is crucial for effective drug delivery to the inner ear. Embodiments, which address one or more of the above challenges will now be described below.

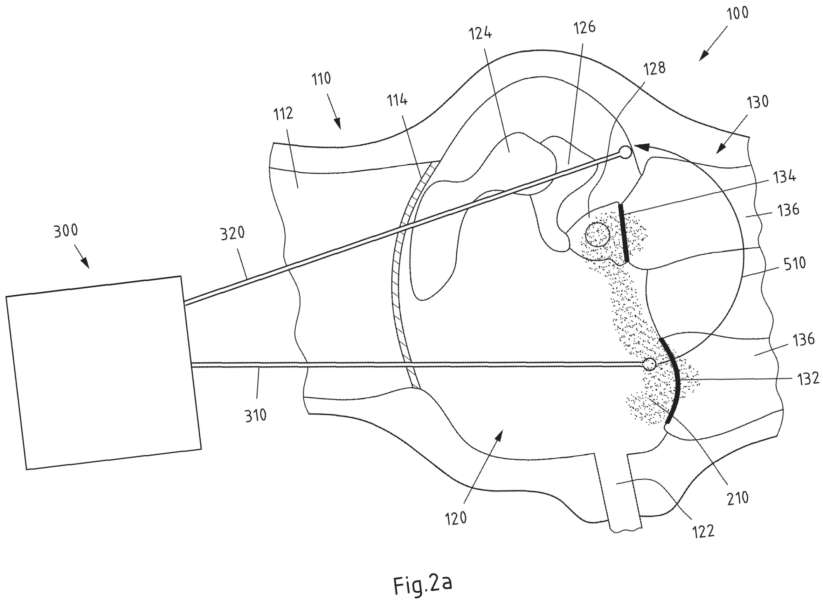

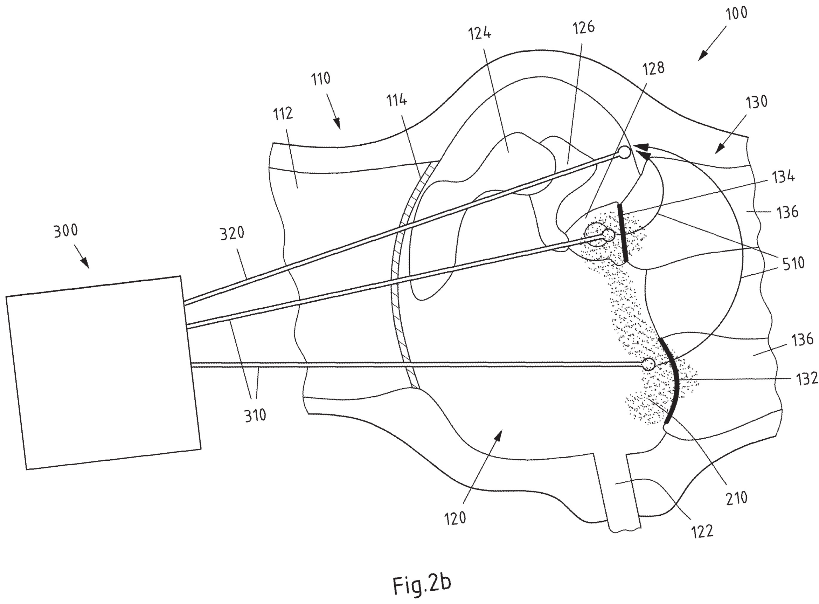

As shown in Figs. 2a and 2b, at least one first electrode 310 (in the following referred to as stimulation electrode) and a second electrode 320 (in the following referred to as return electrode) may be provided in the middle ear space 120. The at least one stimulation electrode 310 and the return electrode 320 may enhance a transfer of the drug 200 through the round window membrane 132 and/or the oval window membrane 134. To enhance the transfer of the drug 200 through the round window membrane 132 and/or the oval window membrane 134, an electrical voltage is provided between the at least one stimulation electrode 310 and the return electrode 320. The electrical voltage causes an electrical current 510 to pass from the at least one stimulation electrode 310 to the at least one return electrode 320. Thereby it is known that the direction of the electrical current 510 depends on the polarity of the electrical voltage and the electrical current 510 may just as well pass from the at least one return electrode 320 to the at least one stimulation electrode 310.

Fig. 2a exemplarily illustrates a first configuration comprising one stimulation electrode 310 arranged close to and in electrical contact with the round window membrane 132 and a return electrode 320 arranged above the oval window membrane 134 in physical contact with tissue of the middle ear space 120.

The stimulation electrode 310 is provided with a distance to the inner ear 130 such that the electrically conductive substance 210 is arranged in part between the inner ear 130 and the stimulation electrode 310. Thereby, the stimulation electrode 310 is provided close to and in electrical contact with the inner ear 130. Further, the stimulation electrode 310 is provided close to and in electrical contact with the round window membrane 132. The stimulation electrode 310 could however equally well be provided close to and in electrical contact with the oval window membrane 134 of the inner ear 130. In particular, the stimulation electrode 310 could be provided close to and in electrical contact to both of the round window membrane 132 and the oval window membrane 134.

The stimulation electrode 310 may be in electrical contact to the inner ear 130 without being in physical contact with the inner ear 130 by being in physical contact with the electrically conductive substance 210. As the electrically conductive substance 210 is electrically conductive and in physical contact with the round window membrane 132 and the oval window membrane 134, an electrical contact to the inner ear 130 can be obtained through the electrically conductive substance 210.

The return electrode 320 may be provided close to and/or in electrical contact with the inner ear 130. In particular, the return electrode 320 may be provided in physical contact with a portion of the inner ear close to the round window membrane 132 and the oval window membrane 134. However, the return electrode 320 may as well be arranged somewhere else in the middle ear space 120, in the outer ear 110, or on the outside of the head comprising the ear. As long as an electrical connection between the return electrode 320 and the stimulation electrode 310 is maintained, a location of the return electrode 320 may be sufficient. On the other hand, by arranging the return electrode 320 close to the round window membrane 132 and/or the oval window membrane 134, a direction and a strength of the electrical current 510 may be controlled more efficiently.

For example, the return electrode 320 may be provided on the promontory (in the middle ear).

The electrical current 510, induced by the electrical voltage, passes at least partly through a part of the inner ear 130. In particular, the electrical current 510 passes at least partly through a part of the round window membrane 132 and/or a part of the oval window membrane 134. While the electrical current 510 is schematically drawn as round arrow in Fig. 2a, the electrical current 510 may flow along a surface of the middle ear space 120 and in particular through one of or both of the round window membrane 132 and the oval window membrane 134.

The flow of the electrical current 510 through the round window membrane 132 enhances or enables a transfer of the drug 200 from the electrically conductive substance 210 to the inner ear 130 through said round window membrane 132. It is thereby possible, that other components of the electrically conductive substance 210 are transferred through the round window membrane 132 as well. However, a transfer of the drug 200 may be a main target.

Analogously, the flow of the electrical current through the oval window membrane 134 enhances or enables a transfer of the drug 200 from the electrically conductive substance 210 to the inner ear 130 through said oval window membrane 134. This is valid also when the stimulation electrode 310 is arranged close to and in electrical contact with the oval window membrane 134 rather than close to and in electrical contact with the round window membrane 132.

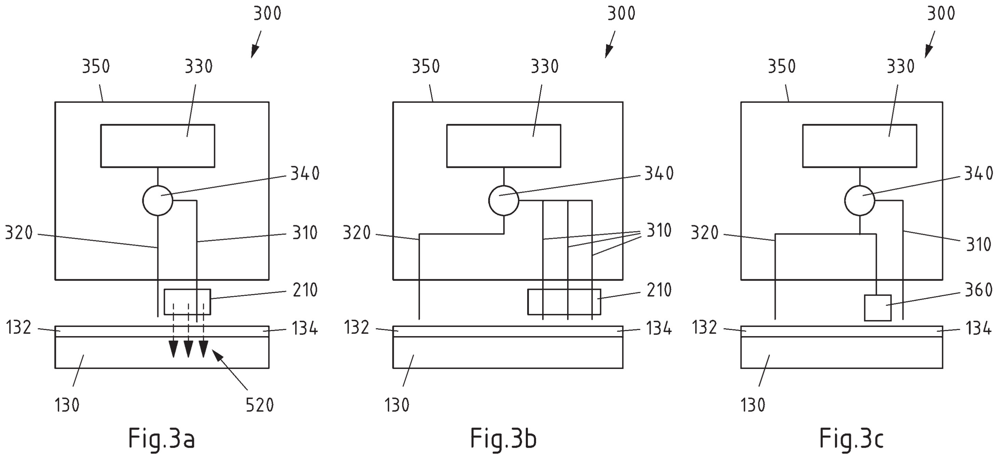

Further, the electrical voltage induces an electrical field 520 (not shown here but illustrated in Fig. 3a by the arrows) between the stimulation electrode 310 and the round window membrane 132. Alternatively, the electrical voltage may induce an electrical field 520 between the stimulation electrode 310 and the oval window membrane 134, in particular when the stimulation electrode 310 is arranged close to and in electrical contact to the oval window membrane 134.

The electrical field 520 may exert a force on charged particles between the stimulation electrode 310 and the round window membrane 132. The force is exerted towards the round window membrane 132 or towards the stimulation electrode 310 depending on a respective charge of the particle and a polarization of the electrical voltage.

As mentioned above, the drug 200 comprises drug particles 220 with an electrical charge. The electrical voltage is provided such that the electrical field 520 exerts a force on the drug particles 220 towards the round window membrane. Thus, the drug particles 220 may move towards the round window membrane 132. A transfer of the drug particles 220 to the inner ear 130 through the round window membrane 132 may thus be enhanced by the electrical field 520. In other words, the device 300 enhances drug penetration by creating a localized electric field 520 that facilitates a movement of charged drug particles 220 across cellular barriers, improving drug distribution within the inner ear 130. By enhancing a diffusion of the drug 200 across the membrane, a chance that the intact drug 200 reaches the target site before it is degraded is increased. Furthermore, a repeated administration-system can be established, allowing for intermittent drug delivery over an extended period in a non-invasive manner. This reduces the need for frequent administrations and improves patient compliance.

Fig. 2b shows another configuration comprising two stimulation electrodes 310, one stimulation electrode 310 arranged close to and in electrical contact with the round window membrane 132 and another stimulation electrode 310 arranged close to and in electrical contact with the oval window membrane 134, and a return electrode 320 arranged above the oval window membrane 134 in physical contact with tissue of the middle ear space 120. Further equally sufficient configurations may include any combination of a single stimulation electrode 310 or return electrode 320 arranged in between the round window membrane 132 and the oval window membrane 134, multiple stimulation electrodes 310 arranged close to and in electrical contact with the round window membrane 132 or the oval window membrane 134, a return electrode close to and in electrical contact with the round window membrane 132 or the oval window membrane 134, a multitude of return electrodes 320 arranged at various locations in the middle ear space 120, or a multitude of stimulation electrodes covering a broad space in the vicinity of the round window membrane 132 and/or the oval window membrane 134.

For the configuration shown in Fig. 2b, each of the two stimulation electrodes 310 may correspond to the stimulation electrode 310 as discussed for Fig. 2a. Thereby, the voltage applied to the two stimulation electrodes 310 is equal or at least of the same polarization. Thus, electrical currents 510 induced by the electrical voltage pass from the respective stimulation electrode 310 to the return electrode 320.

Further, the electrical voltage induces an electrical field 520 between the round window membrane 132 and the stimulation electrode 310 arranged close to the round window membrane 132, and the electrical voltage induces an electrical field 520 between the oval window membrane and the stimulation electrode 310 arranged close to the oval window membrane 134. Thus, both electrical fields 520 may exert a force on charged drug particles 220 towards the inner ear 130.

In particular, a respective electrical field 520 may exert a force on charged particles between a respective stimulation electrode 310 and the round window membrane 132, while the respective other electrical field 520 may exert a force on charged particles between the respective other stimulation electrode 310 and the oval window membrane 134. The electrical voltage is provided such that the electrical fields 520 exerts a force on the drug particles 220 towards the round window membrane or towards the oval window membrane, respectively. Thus, the drug particles 220 may move towards the round window membrane 132 or towards the oval window membrane 134, respectively. A transfer of the drug particles 220 to the inner ear 130 through the round window membrane 132 and through the oval window membrane 134 may thus be enhanced by the electrical fields 520 simultaneously.

Figs. 3a-3c schematically show exemplary devices 300 according to the application. In particular, the shown devices 300 may correspond to devices shown and discussed in Figs. 1, 2a and 2b. The devices 300 comprise a housing 350, supporting a current controller 330 and a current generator 340. The current controller 330 is configured to control the current generator 340 to generate an electrical voltage. The current generator 340 applies the electrical voltage to the stimulation electrodes 310 and return electrodes 320. An electric stimulation applied by the device 300 can be either AC or DC, of varying current strength (common strengths range up to 0.5 mA), periodicity, polarity (depending on the drug particle's charge and intended mechanism of action), and durations (typically few minutes).

The stimulation electrodes 310 are arranged in contact with the electrically conductive substance 210. The electrically conductive substance 210 and the tips of the stimulation electrodes 310 are arranged close to and/or in electrical contact with the inner ear 130. The round window membrane 132 and the oval window membrane 134 are shown as a single layer separating the inner ear 130 from the middle ear space 120 (not shown here) for illustrative purposes. The return electrodes 320 are arranged close to the inner ear 130. A gap illustrated between the round window membrane 132 and/or oval window membrane 134 is just exemplary and not for scale. The stimulation electrodes 310 and the return electrodes 320 may be in direct physical contact with the round window membrane 132 and/or the oval window membrane 134. Alternatively, the stimulation electrodes 310 and the return electrodes 320 may be spatially separated from the round window membrane 132 and/or the oval window membrane 134.

Fig. 3a shows an exemplary device 300 comprising one stimulation electrode 310 and one return electrode 320. The return electrode 320 is arranged close to the stimulation electrode 310. The current generator 340 is configured to apply an electrical voltage to the stimulation electrode 310 and the return electrode 320. Thereby, the applied electrical voltage induces an electrical field 520, which is illustrated in Fig. 3a as arrows. The arrows represent a direction of force applied to electrically charged drug particles 220 arranged in the electrically conductive substance 210 by the electrical field 520.

At the same time the arrows may thus represent a direction of motion of the drug particles 220 from the electrically conductive substance 210 through the round window membrane 132 and/or the oval window membrane 134 into the inner ear 130. In the inner ear 130, the drug particles 220 may unfold a therapeutic effect to components of the inner ear 130.

Fig. 3b shows another exemplary device 300 comprising three stimulation electrodes 310 and one return electrode 320. The number of stimulation electrodes 310 is merely exemplary and chosen to avoid complex illustrations. A higher number, such as e.g. ten electrodes, may be equally applicable. Due to the device 300 comprising multiple (in Fig. 3b three) stimulation electrodes, a distribution of the electrical field 520 can be increased or modulated to further enhance a force applied to the drug particles 220. The return electrode 320 is arranged at a larger distance from the stimulation electrodes 310, compared to a distance between the stimulation electrode 310 and the return electrode 320 as shown in Fig. 3a. Thus, the electrical current 510 (not shown in Figs. 3a-3c) will pass a further distance through the round window membrane 132 and/or the oval window membrane 134 from the stimulation electrodes 310 to the return electrode 320.

Fig. 3c shows a further exemplary device 300 comprising one stimulation electrode, one return electrode and a sensor or chamber 360. The sensor or chamber 360 is configured to collect, sample and/or detect fluids from the inner ear 130. The sensor or chamber 360 is arranged close to the round window membrane 132 and/or the oval window membrane 134. Furthermore, the sensor or chamber 360 is connected to the current generator 340. In particular, the sensor or chamber 360 is connected to the return electrode and a voltage provided by the current generator 340 between the return electrode and the stimulation electrode 310 is also provided between the sensor or chamber 360 and the stimulation electrode 310.

When fluids are transferred from the inner ear 130 to the middle ear space 120 through the round window membrane 132 and/or the oval window membrane 134, the sensor or chamber collects or samples said fluids from the inner ear 130. Thus, information on a state of the inner ear provided by said fluids may be obtained.

In view of the above figures, a system according to the application may comprise or correspond to a combination of the syringe 230 shown in Fig. 1, and a device 300 according to any of Figs. 2a-b and 3a-c. Thus, the described system is for the use in a method for delivering drugs 200 to an inner ear 130. Further, the device 300 is for the use in a method for delivering drugs 200 to an inner ear 130.

The described approach and embodiments in particular allow for transporting molecules of a substance of interest into the inner ear, e.g. across the oval window membrane and/or round window membrane, by increasing the permeability of the membrane with an electric current from a treatment device and utilizing a driving force to move the molecule of interest through the permeabilized membrane. As described herein, the device may consist of a current controller and generator which is connected to one or more stimulation electrodes and one or more return electrodes. The applied current floats through the tissue between the stimulation and return electrode. The electric stimulation applied can be either AC or DC, of varying current strength (common strengths range up to 0.5 mA), periodicity, polarity (depending on the drug's charge and intended mechanism of action), and durations (typically few minutes).

The use of iontophoresis to deliver drugs to the inner ear may in particular solve several problems associated with traditional drug delivery methods:
1. Poor drug penetration: Some drugs have limited penetration from the middle ear into the inner ear when administered through traditional intra-tympanic administration routes. For example, certain antibiotics may not adequately reach the cochlea, where they are needed to treat infections like labyrinthitis. Another example is the poor penetration of neurotrophic drugs for protection or regeneration of inner ear structures, using for example growth factors The approaches described herein utilize iontophoresis and can enhance drug penetration by creating a localized electric field that facilitates the movement of charged drug molecules across cellular barriers, improving drug distribution within the inner ear.
2. Drug stability and degradation: the approaches described herein utilize iontophoresis and can enhance diffusion of the drug across membrane, thereby increasing the chance that the intact drug reaches the target site before it is degraded. The delivery method can be designed such that it does not alter the chemical properties of the drug.
3. Systemic side effects: Systemic administration of drugs can lead to various side effects throughout the body, especially if high drug doses are required. By using the approaches described herein with iontophoresis, the drug can be localized to the inner ear, minimizing the risk of systemic side effects. This targeted drug delivery approach enhances the therapeutic benefit while reducing the potential for adverse reactions in other organs or tissues.
4. Chronic conditions: Many inner ear disorders, such as Ménière's disease or tinnitus, are chronic conditions that require long-term drug treatment. With the approaches described herein utilizing iontophoresis, a repeated administration-system can be established, allowing for intermittent drug delivery over an extended period in a non-invasive manner. This reduces the need for frequent administrations and improves patient compliance.

As described, the approaches described herein may also be used together with a conductive gel formulation that can enhance transport of the molecule across the oval window membrane and/or round window membrane and into the inner ear.

The approached described herein may also include a chamber to sample fluids from the inner ear, because of the increased permeability membrane of the round window membrane and/or oval window membrane. Such fluid samples could be used for diagnostic purposes or for pharmacokinetic profiling of therapeutics delivered to the inner ear.

### Items:

1. A method for supporting delivery of a substance (200) to an inner ear (130), the method comprising:
   - providing at least one first electrode (310) in a middle ear space (120) close to and/or in electrical contact with the inner ear (130);
   - providing at least one second electrode (320); and
   - providing an electrical voltage between the at least one first electrode (310) and the at least one second electrode (320), so as to support delivery of the substance (200), provided in the middle ear space, into the inner ear (130).
2. The method according to item 1, wherein the substance (200) is provided in an electrically conductive substance (210).
3. The method according to item 2, wherein the electrically conductive substance (210) is or comprises a conductive gel or a conductive solution.
4. The method according to item 2 or 3, wherein the electrically conductive substance (210) is arranged at least in part between the inner ear (130) and the at least one first electrode (310).
5. The method according to any of items 1 to 4, wherein at least one of the at least one first electrode (310) is provided close to and/or in electrical contact with a round window membrane (132) and/or an oval window membrane (134) of the inner ear (130).
6. The method according to any of items 1 to 5, wherein at least one of the at least one first electrode (310) is provided close to and/or in electrical contact with the round window membrane (132) and at least a second one of the at least one first electrode (310) is provided close to and/or in electrical contact with the oval window membrane (134).
7. The method according to any of items 1 to 6, wherein the at least one second electrode (320) is provided close to and/or in electrical contact with the inner ear (130).
8. The method according to any of items 1 to 7, wherein the electrical voltage causes an electrical current (510) to pass from the at least one first electrode (310) to the at least one second electrode (320), in particular wherein the electrical current (510) passes at least partly through a part of the inner ear (130), in particular wherein the electrical current (510) passes at least partly through a part of the round window membrane (132) and/or a part of the oval window membrane (134).
9. The method according to any of items 1 to 8, wherein the electrical voltage induces an electrical field (520) between at least one of the at least one first electrode (310) and the round window membrane (132) and/or between at least one of the at least one first electrode (310) and the oval window membrane (134).
10. The method according to any of items 1 to 9, wherein the substance (200) comprises particles (220) with an electrical charge, in particular wherein the electrical voltage is provided such that the electrical field (520) exerts a force on the particles (220) towards and/or through the round window membrane (132) and/or the oval window membrane (134).
11. The method according to any of items 1 to 10, further comprising:
   - providing a sensor or chamber (360) close to the round window membrane (132) and/or the oval window membrane (134); and
   - receiving fluids from the inner ear (130) at the sensor or chamber (360).
12. A device (300), in particular configured to perform a method according to any of items 1 to 11, the device (300) comprising:
   - at least one first electrode (310);
   - at least one second electrode (320);
   - means to provide the at least one first electrode (310) in the middle ear space (320) close to and/or in electrical contact with the inner ear (130);
   - means to provide the at least one second electrode (320); and
   - means to provide an electrical voltage between the at least one first electrode (310) and the at least one second electrode (320), so as to support delivery of a substance (200), provided in the middle ear space, into the inner ear (130).
13. The device (300) according to item 12, further comprising at least one of:
   - a sensor or chamber (360), configured for receiving fluids from the inner ear (130); and
   - means to provide the sensor or chamber (360) close to the round window membrane (132) and/or the oval window membrane (134).
14. The device (300) according to item 12 or 13, for the use in a method for supporting delivery of a substance (200) to an inner ear, in particular a method according to any of claims 1 to 11.
15. A system, in particular for use in or configured to perform a method for supporting delivery of a substance to an inner ear, in particular a method according to any of claims 1 to 11, the system comprising
   - the device (300) according to any of items 12 to 14 and
   - means (230) for providing a substance (200) into a middle ear space (120).

It is intended that the structural features of the devices described above, either in the detailed description and/or in the claims, may be combined with steps of the method, when appropriately substituted by a corresponding process.

As used, the singular forms "a," "an," and "the" are intended to include the plural forms as well (i.e. to have the meaning "at least one"), unless expressly stated otherwise. It will be further understood that the terms "includes", "comprises", "including", and/or "comprising", when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element, but an intervening element may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method are not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects. Reference to an element in the singular is not intended to mean "one and only one" unless specifically so stated, but rather "one or more." Unless specifically stated otherwise, the term "some" refers to one or more.

Accordingly, the scope should be judged in terms of the claims that follow.

## Claims

1. A device (300) comprising:
- a first electrode (310), and
- a second electrode (320),
- wherein the first electrode (310) is configured to be located in a middle ear space in electrical contact with an inner ear (130),
- wherein the second electrode (320) is configured to be located in the middle ear space, and
- wherein the device further comprises a controller for controlling a current generator configured to generate an electrical voltage between the first electrode (310) and the second electrode (320), so as to support delivery of a substance (200), provided in the middle ear space, into the inner ear (130).

2. The device according to claim 1, wherein the first electrode is a stimulation electrode.

3. The device according to any one of the preceding claims, wherein the second electrode is a return electrode.

4. The device (300) according to any one of the preceding claims, further comprising:
- a sensor or chamber (360) configured to receive fluids from the inner ear (130), and where the sensor or chamber (360) is configured to be located at the round window membrane (132) and/or the oval window membrane (134).

5. The device according to claim 4, wherein the sensor or chamber is connected to the current generator.

6. The device according to any one of claims 4-5, wherein the sensor or chamber is connected to the return electrode.

7. The device according to any one of claims 4-6, wherein the electrical voltage generated by the current generator between the return electrode and the stimulation electrode is also provided between the sensor or chamber and the stimulation electrode.

8. The device according to any one of claims 2-7, wherein the device comprises a further stimulation electrode, and where the electrical voltages generated by the current generator between each of the stimulation electrodes and the return electrode are of similar polarization.

9. The device according to any one of the claims 2-8, wherein the device comprises a plurality of stimulation electrodes.

10. The device according to any one of the preceding claims, wherein the device further comprises a ground electrode.

11. The device according to any one of the preceding claims, wherein the substance (200) is provided in an electrically conductive substance (210).

12. The device according to any one of the preceding claims, wherein the electrically conductive substance (210) is or comprises a conductive gel or a conductive solution.

13. The device according to any one of the preceding claims, wherein the device is configured to provide an electrical current (510) to pass from the first electrode (310) to the second electrode (320) at least partly through a part of the round window membrane (132) and/or a part of the oval window membrane (134).

14. The device according to any one of the preceding claims, wherein the device is configured to provide an electrical voltage inducing an electrical field (520) between at least one of the first electrode (310) and the round window membrane (132) and/or between at least one of the first electrode (310) and the oval window membrane (134).

15. The device according to any one of the preceding claims, wherein the substance (200) comprises particles (220) with an electrical charge such that the electrical field (520) exerts a force on the particles (220) towards and/or through the round window membrane (132) and/or the oval window membrane (134).

16. A system for use in or configured to support delivery of a substance to an inner ear, the system comprising
- the device (300) according to any of the preceding claims, and
- a syringe (230) for providing a substance (200) into a middle ear space (120).
